# EUROPÄISCHE PATENTANMELDUNG

(11) **EP 3 705 562 A1**
(43) Veröffentlichungstag der Anmeldung: **09.09.2020**
(21) Anmeldenummer: 19000451.5
(22) Anmeldetag: 07.10.2019
(51) Int. Cl.: C12M 1/00, C12M 1/12, C12N 5/00

(54) **ZELLKULTURTRÄGER FÜR BIOREAKTOREN**

(30) Priorität: 06.03.2019 DE 102019001604
(71) Anmelder: Zellwerk GmbH, 16727 Oberkrämer (DE)
(72) Erfinder: Hoffmeister, Hans, D- 32549 Bad Oeynhausen (DE)

(57) **Zusammenfassung**

Die Erfindung betrifft einen Zellkulturträger für Bioreaktoren zur Isolierung, Vermehrung und Ernte von Zellen, insbesondere von NK- Zellen, T-Lymphozyten und tumorinfiltrierende Lymphozyten (TIL) und mesenchymalen Stammzellen aus Knochenmark, wobei der Träger eine für die jeweilige Zellart spezifische Beschichtung aufweist (adhäsiv; Rezeptor/Ligand; Ko-Rezeptor usw.).

## Beschreibung

Die Erfindung betrifft einen Zellkulturträger für Bioreaktoren zur Isolierung, Vermehrung und Ernte von Zellen, insbesondere von NK- Zellen, T-Lymphozyten und tumorinfiltrierende Lymphozyten (TIL) und mesenchymalen Stammzellen aus Knochenmark, wobei der Träger eine für die jeweilige Zellart spezifische Beschichtung aufweist (adhäsiv; Rezeptor/Ligand; Ko-Rezeptor usw.). Aus dem Stand der Technik sind eine Vielzahl von Zellkulturträgern für die Züchtung und Vermehrung von Zellen bekannt. Zur Kultivierung von Zellen in hoher Dichte ist es erforderlich, dass sich diese an ein künstliches oder natürliches Substrat anheften oder in Berührung mit der Oberfläche des Zellkulturträgers geraten, damit das Überleben, die Vermehrung der Zellen und/oder das bevorzugte Wachstum eines enthaltenen Phenotyps sichergestellt wird. Die Produktion einer gewünschten Zellart erfordert in erster Linie, dass die Substratoberfläche eine besondere/spezifische heraussagende Zelladhäsion oder Zellberührung ermöglicht und die Fähigkeit zur Unterstützung des Zellwachstuns besitzt.

Die DE 42 06 585 A1 offenbart eine Vorrichtung zur Behandlung von Zellkulturen, insbesondere von Hepatozyten, auf plattenartigen Zellkulturträger, wobei wenigstens ein Teil der Oberflächen der Zellkulturträger gasdurchlässig ist. Auf dem Zellkulturträger ist eine Kollagenschicht aufgetragen, auf der oder in der die Zellkultur angeordnet ist. Mit geringem Abstand über der Kollagenschicht ist der nächste Zellkulturträger angeordnet. In den Zwischenraum zwischen der Kollagenschicht und dem nächsten Zellkulturträger ist Kulturmedium einleitbar.

In der DE 69 022 778 T2 wird ausgeführt, dass für die Adhäsion an Zellen hochmolekulare Materialien erforderlich sind, deren Oberflächen mit Niedertemperaturplasma, Sputtern, UV-Licht, Elektronenstrahlen behandelt sind und die mit einem monomolekularen, anhaftender Film bedeckt sind, der mit Zelladhäsionsfaktoren und Zellwachstumsfaktoren behandelte Materialien enthält.

Dabei sind zelladhäsive Filme aus Collagen, Fibronektin, Vitronektin und Laminin bekannt.

Die Erfindung gemäß DE 69 115 928 T2 betrifft eine Unterlage für die Kultivierung von haftungsabhängigen Zellen (CAD) in Form von Mikroträgem aus einem biokompatiblen Material. Erfindungsgemäß wird eine Unterlage für die Kultivierung von CAD in Form von Mikroträgern aus einem biokompatiblen Material bereitgestellt, wobei die Mikroträger eine zweidimensionale Geometrie besitzen und zwei aneinander liegende Flächen zur Anhaftung mit einer Stärke von 35 um oder weniger aufweisen. Auf jeder der beiden Flächen können sich die Zellen anheften und entwickeln, ohne dass ein Eindringen der Zellen zwischen die beiden Flächen möglich ist. Die Mikroträger bestehen aus einem Polymermaterial, beispielsweise ein Polymer mit zahlreichen aromatischen Gruppen und insbesondere Polystyrol, aber auch Scheibchen aus hydrophilem Material, wie Cellophan oder Poly-D-hydroxybutyrat. Die Mikroträger sind biokompatibel, wobei unter Biokompatibilität verstanden wird, dass die CAD an den Flächen der erfindungsgemäßen Mikroträger haften und sich dort vermehren können. Zu diesem Zweck ist vorgesehen, dass die beiden Flächen der erfindungsgemäßen Mikroträger mit einer dünnen Metallschicht überzogen sind. Dieser Überzug hat dabei eine Titanbasis, aber auch Platin wird in Betracht gezogen

Die DE 10 2004 053 164 A1 betrifft beschichtete Mikroträger für die Kultivierung tierischer und humaner Zellen vorwiegend in mit Nährmedium gefüllten durchmischten Gefäßen, wie beispielsweise Festbett- oder Wirbelbettreaktoren. Der Mikroträger besteht aus einem partikulären anorganischen porösen oder nichtporösen Matrixmaterial, das zu einem Stück verfestigt ist, und einer zellkulturkompatiblen, Hydrolyse-stabilen Epoxid-Amin-Polymer-Beschichtung, die diesem Stück aufgelagert sind. Die Größe der Partikel liegt in einem Bereich von 100 bis 1000 um, vorzugsweise in einem Bereich von 180 bis 590 um. Geeignete Matrixmaterialien für die Mikroträger sind beispielsweise Glas, Keramiken, Silicagel, Gips, Metalloxide, Metallsilicate oder Tricalciumphosphate. Besonders vorteilhaft als Trägermaterial eignet sich ein Glas aus dem System Si0₂-CaO-Na₂0-MgO-K₂0-Al₂0₃-Fe₂0₃. Das als Beschichtung des anorganischen Matrixmaterials verwendete vernetzte Epoxid-Amin-Polymer wird aus einer oder mehreren Epoxidkomponenten und einem oder mehreren Amin-Komponenten hergestellt.

In der DE10 2005 035 434 A1 werden Zellkulturträger offenbart, auf denen Zellen effizient anhaften und wachsen und von denen die gewachsenen Zellen einfach gelöst werden. Die Zellkulturträger enthalten jeweils ein körniges Grundmaterial und eine Deckschicht, die die Oberfläche des Grundmaterials abdeckt. Die Deckschicht besteht hauptsächlich aus einem Calciumphosphat-basierten Apatit, bei dem ein Teil des Calciums fehlt. Solche Zellkulturträger werden in Zellkulturen verwendet, in denen die Zellen auf den Oberflächen der Zellkulturträger haften und dort wachsen. Insbesondere werden diese Zellkulturträger in dreidimensionalen Kulturen hoher Dichte (Suspensionskulturen) verwendet. Der Ca-Mangel in dem Calciumphosphat-basierten Apatit liegt vorzugsweise in einem Bereich von 1 bis 30 Mol-%.

Die Erfindung gemäß WO 2010/136136 A3 betrifft ein Verfahren zur Herstellung eines beschichteten Zellkulturträgers zur In-vitro-Kultivierung von Stammzellen, insbesondere zur Kultivierung von mesenchymalen Stammzellen, wobei eine Lösung, die einen Polyurethanharnstoff umfasst, auf einen Zellträger aufgebracht und getrocknet wird. Der Polyurethanharnstoff wird zuvor durch Umwandlung von mindestens einer Polycarbonatpolyol-Komponente, mindestens einer Polyisocyanatkomponente und mindestens einer Diamino-Komponente hergestellt. Antioxidantien oder Pigmente, aber auch Griffhilfsmittel, Farbstoffe, Mattierungsmittel, UV-Stabilisatoren, Lichtstabilisatoren, Hydrophilierungsmittel, Hydrophobierungsmittel und/oder Verlaufshilfsmittel können als Zusätze eingesetzt werden. Beschichtet werden vielerlei Substrate wie Glas, Siliciumwafer, Metalle, Keramiken und Kunststoffe. Bevorzugt ist die Beschichtung von Zellkulturträgern aus Glas, Siliciumwafer, Kunststoff oder Metallen. Als Metalle werden beispielsweise medizinischer Edelstahl und Nickel-Titan-Legierungen genannt werden. Als Polymermaterialien, aus denen der Zellkulturträger aufgebaut ist, werden Polyamid; Polystyrol; Polycarbonat; Polyether; Polyester; Polyvinylacetat; natürliche und synthetische Kautschuke; Blockcopolymere aus Styrol und ungesättigten Verbindungen wie Ethylen, Butylen und Isopren; Polyethylen oder Copolymeren aus Polyethylen und Polypropylen; Silikon; Polyvinylchlorid (PVC) und Polyurethanen eingesetzt. Zur besseren Haftung der erfindungsgemäßen Polyurethane auf dem Zellkulturträger können als Untergrund vor dem Auftragen dieser Beschichtungsmaterialien noch weitere geeignete Beschichtungen aufgetragen werden. Besonders bevorzugt ist die Beschichtung von Glas oder Siliciumwafern zur Herstellung von Zellkulturträgern.
Als Lösemittel für die Herstellung der Polyurethanharnstoff-Lösungen kommen werden alle denkbaren Lösemittel und Lösemittelgemische wie Dimethylformamid, N-Methylacetamid, Tetramethylharnstoff, N-Methylpyrrolidon, aromatische Lösemittel wie Toluol, lineare und cycüsche Ester, Ether, Ketone und Alkohole in Frage. Beispiele für Ester und Ketone sind Ethylacetat, Butylacetat, Aceton, v-Butyrolacton, Methylethylketon und Methylisobutylketon eingesetzt. Bevorzugt sind Mischungen aus Alkoholen mit Toluol. Beispiele für Alkohole, die gemeinsam mit Toluol verwendet werden können, sind Ethanol, n-Propanol, iso-Propanol und 1-Methoxy-2-propanol.
Die Beschichtungen aus den Polyurethanharnstofflösungen können mittels verschiedener Verfahren auf den Zellkulturträger aufgebracht werden. Geeignete Beschichtungstechniken sind beispielsweise Rakeln, Drucken, Transferbeschichten, Sprühen, Spincoating oder Tauchen.

In der WO 2016/202329 A1 wird ein bioaktives Beschichtungsmaterial zur Beschichtung von Kunststoffmaterialien für Zellkulturen, vorzugsweise Kunststoffeinwegmaterialien, zum Beispiel aus Polystyren (TCP) als Kunststoff offenbart, insbesondere ein zellinstruktives Beschichtungsmaterial für Zellkulturmaterialien aus Kunststoff, zum Beispiel Polystyren. Das bioaktives Beschichtungsmaterial umfasst ein Polymerkonjugat aus jeweils einem polymeren Ankermolekül mit oberflächenaktiven Ankergruppen einerseits und aus jeweils einem oder mehreren biologisch aktiven Molekülen andererseits, wobei das Ankermolekül ein amphiphiles Molekül mit einem hydrophoben Teil aus Styren-, Methacrylsäure- oder Isobuteneinheiten und einem hydrophilen Teil aus Maleinsäureanhydrid- oder Maleinsäure-Einheiten ist.

Die EP 1 270 533 A2 beschreibt eine Trägermaterial für Zellkulturträger aus keramische Materialien für eine verbesserte Unterstützung für die Kultur von verankerungsabhängigen eukaryotischen Zellen und prokaryotischen Zellen , die eine kristalline Kernoxidkeramik umfassen, die mit einer amorphen polyanionischen intergranularen Phase dispergiert ist. Das keramisches Material besteht aus einem Oxid der kristallinen Kernoxidkeramik vorzugsweise aus der Gruppe bestehend aus Zirkonium (IV) oxid, Aluminiumoxid, Silizium (IV) oxid, Titan (IV) oxid, Hafnium (IV) oxid, Cer (IV) oxid und Mischungen davon ausgewählt. Die polyanionische Verbindung in der amorphen intergranularen Phase ist aus der Gruppe bestehend aus Phosphaten, Manganaten, Molybdaten, Wolframaten oder Mischungen davon ausgewählt.

Mit dem aus dem Stand der Technik dargestellten Zellkulturträger aus einem Trägermaterial mit einer Beschichtung aus zelladhäsivem Material lassen sich Zellen nur in einer Menge isolieren, vermehren und ernten, die für eine Zelltherapie ungeeignet ist, wobei die Zeilen auch eine geringe zytotoxische Wirkung aufweisen.

Aufgabe der Erfindung ist es, einen Zellkulturträger zu entwickeln, mit dessen Hilfe Zellen, insbesondere NK- Zellen sowie autologe, tumorinfiltrierende Lymphozyten und autologe T- Lymphozyten in ausreichender Menge isoliert, ex vivo spezifisch aktiviert sowie vermehrt und geerntet werden können. Die geernteten Immunzellzellen werden als Suspension lebender Zellen in vivo für eine Therapie gegen Tumorzellen verwendet, wobei die Zellen eine hohe spezifische Toxizität besitzen gegen das jeweils einmalige Gemisch an mutierten Lymphozyten eines Patienten-individuellen Tumors bzw. seiner Metastasen. Lymphozyten ie jeweils individuellen Tumorzellen

Die Aufgabe wird durch einen Zellkulturträger (20) gelöst, der aus einem hydrophilen, mit Niedertemperatur- Plasma behandelten Trägerkörper (21) mit einer zelladhäsiven Beschichtung (22) besteht, wobei in die zelladhäsive Beschichtung (22) aktivierende Antikörper eingearbeitet sind.
Der Trägerkörper (21) besteht aus Kunststoff, Glas, Metall oder keramische Materialien.
Die zelladhäsive Beschichtung (22) besteht aus Fibronektin, RetroNectin, Collagen, Gelatine, Laminin, Chinitin und/oder Kombinationen davon. Als aktivierende Antikörper werden Immunglobuline, β-Lymphozyten, Anti CD 16, Anti CD3, Anti CD28, Anti137, Anti CD 39, Anti CD103 und/oder Kombinationen davon in die zelladhäsive Beschichtung eingearbeitet.

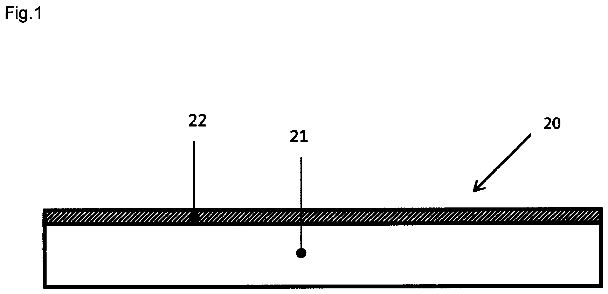

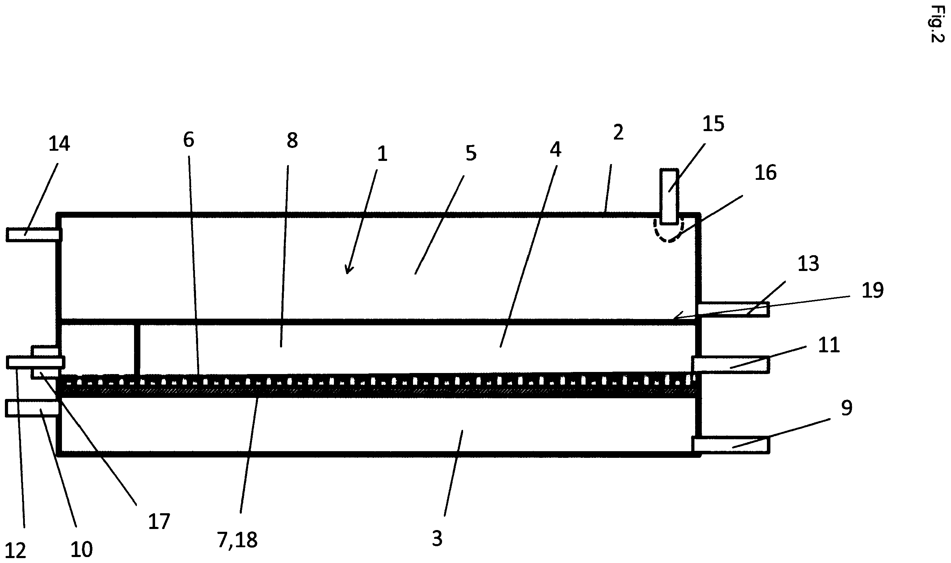

Der erfindungsgemäße Zellkulturträger kann in Festbett-Bioreaktoren, Fließbett-Reaktoren, Walzen- Bioreaktoren, Hohlfaser- Bioreaktoren oder Mäander-Bioreaktoren, die mit einer zelladhäsiven oder Beschichtung mit darin enthaltenen aktivierenden Antikörper zur Isolierung und Vermehrung von Zellen eingesetzt werden. Mit dem erfindungsgemäßen Zellkulturträger gelingt es auch , aus kleingeschnittenen Gewebestücken bestimmte Zellen sowohl in das im Bioreaktor befindliche Medium auswachsen zu lassen, als auch die Zellen gleichzeitig dynamisch und stressfrei zu expandieren, differenzieren, aktivieren, stimulieren und dann von Geweberesten abzutrennen. Insbesondere können auf diese Weise NK-Zellen, tumorinfiltrierende autologe T- Lymphozyten (TIL) aus Tumorgewebe, Metastasengewebe, deren Umgebungsgewebe und aus Lymphknoten, die von Tumorzellen befallenen sind, aber auch die Gesamtfraktion oder einzelne Zellarten der hämatopoetischen Zellen, hämatopoetischen Stammzellen, hämatopoetischen Progenitorzellen, Immunzeifen darin, gewonnen werden, wobei der Bioreaktor jeweils im Perfusionsbetrieb arbeitet.
Die Erfindung wird nun näher an Beispielen erläutert, wobei Fig. 1 eine Skizze des Zellkulturträgers (20) und die Fig. 2 eine Skizze des Mäander- Bioreaktors aus
DE 20 2018 004 857.7 mit der Oberseite (18) der Bodenfläche (6) als Zellkulturträger (20) zeigt, wobei
- 1: Bioreaktorgefäß
- 2: Deckel
- 3: Unterlay- Kammer
- 4: Mänder- Perfusions- Kammer
- 5: Overiay- Kammer
- 6: Bodenplatte mit Beschichtung (22) als Zellkulturträger (20)
- 7: Folie
- 8: Trennwand
- 9: Zuführung Unterlay- Atmosphäre
- 10: Abführung Unterlay-Atmosphäre
- 11: Zuführung Kulturmedium
- 12: Abführung Kulturmedium
- 13: Zuführung Overlay- Atmosphäre
- 14: Abführung Overlay-Atmosphäre
- 15: Auslaufstutzen
- 16: Siebgewebe
- 17: Überlauf für Zellbrühe
- 18: Oberseite der Bodenplatte (6)
- 19: Gerinne
- 20: Zellkulturträger
- 21: Trägerkörper
- 22: Beschichtung
bedeuten.

### Ausführungsbeispiel 1

Der Mäander-Perfusions-Bioreaktor gemäß DE 20 2018 004 857.7 besteht aus einen rechteckigen Bioreaktorgefäß (1) aus vorzugsweise klarem Polymermaterial, das mit einem Deckel (2) steril verschlossen ist. Das Bioreaktorgefäß (1} ist in drei übereinander angeordneten Kammern (3, 4, 5) unterteilt, wobei die unterste Kammer (3) als Unterlay- Kammer, die über der Unterlay-Kammer (3) angeordnete Kammer (4) als Mäander- Perfusions- Kammer und die über der Mäander- Perfusions-Kammer (4) angeordnete Kammer (5) als Overlay- Kammer ausgebildet sind. Die Unterlay- Kammer(3) und die Mäander- Perfusions- Kammer (4) sind durch eine gelochte Bodenplatte (6) mit einer unten an der gelochten Bodenplatte (6) angeordneten semipermeabten Folie (7) voneinander getrennt. Auf der Oberseite (18) der Bodenplatte (6) sind streifenförmigen unterteilenden und eine mäanderförmige Durchströmung der Mäander- Perfusions-Kammer (4) mit Gasen und Medium erzwingenden Trennwänden (8) angeordnet, wobei der Abstand (A) der Trennwände (8) voneinander sowie von den Seiten- und Stirnwänden der Mäander-Perfusions- Kammer (4) so gewählt ist, dass sich in dem Stromfaden, in dem durch die Trennwände (8) gebildeten Gerinne, eine gemittelte laminare Oberströmung mit einer Froudezahl < 0,005 bildet. Dieser Abstand (A) beträgt 2 bis 25 mm, bevorzugt 4 bis 6 mm.

Die Bodenplatte (6) der Mäander- Perfusions- Kammer (4) ist als Trägerkörper (21) eines Zellkulturträgers (20) ausgebildet und ist auf der in Richtung der Mäander-Perfusios- Kammer (4) zeigende Oberseite(18) mit einer zelladhäsiven Beschichtung (22) versehen. Dazu wird in Wasser gelöstes Fibronektin gemischt mit Anti-CD3 als aktivierendem Antikörper in die durch die Trennwände (8) gebildeten Gerinne (19) des Mäander Bioreaktors verbracht, gleichmäßig durch Kippen des Mäander Bioreaktors verteilt und ausgetrocknet.

An der Unterlay-Kammer (3) sind in unterschiedlicher Höhe Zu- und Abführungen (13,14) für eine geregelte Durchströmung mit Gasen, insbesondere von Luft, gemischt mit unterschiedlichen Volumen-Prozent an Sauerstoff (5% bis 50%, bevorzugt 10 bis 25% Volumenprozent). Der Sauerstoff gelangt dann durch Diffusion durch die semipermeable Folie (7) und durch die gelochte Bodenplatte (6) in die Mäander- Perfusions- Kammer (4), wo dann das

Kulturmedium bei der Durchströmung der Mäander- Perfusions- Kammer (4) durch Perfusion mit O₂ versorgt wird, Die Mäander- Perfusions- Kammer (4) ist mit Zu- und Abführungen für Kulturmedien (11,12) versehen, wobei das Kulturmedium aus einem CellGrow-Medium besteht, das mit einem spezifischen Gemisch aus AB Humanserum, Cytokinen, Antikörpern und irradiierten humanen Feederzellen zeitweilig supplementiert ist. Diesem Kulturmedium werden die während einer Operation vom Patienten entnommenen und zerkleinerten autologen Tumor-Gewebestücke in einer Größe von 1 bis 2 mm3 zugegeben und mit dem Kulturmedium in die Mäander-Perfusions-Kammer (4) zugeführt. Die zerkleinerten Gewebestücke werden im Bioreaktor gleichmäßig verteilt und im Perfusionsbetrieb kultiviert, wobei das Kulturmedium über die in die Overlay-Kammer (5) über die Zu- und Abführungen (13,14) mit der entsprechenden Overiay-Atmosphäre geregelt versorgt wird.

In einem Kuitivierungslauf über 7 bis 14 Tage wachsen normalenweise TIL in größerer Menge aus den Gewebestücken aus. Über den mit Siebgewebe (16) versehenen Auslassstutzen (15) der Overlay-Kammer (5) des Bioreaktorgefäßes (1) werden die TIL als Filtratvon den Geweberesten abgetrennt.

Mit zunehmender Zellzahl im Verlauf der Vermehrung der Zellmenge (TIL oder andere Zellen) wird die Zufuhr von frischem Medium kontinuierlich erhöht, wobei das erhöhte Zuführen von frischem Medium durch einen entsprechenden Algorithmus automatisch gesteuert wird. Die Bodenströmung verbleibt dabei nahe einer Froudezahl 0. Durch diese langsame Bewegung des Kulturmediums wird eine Aufwirbelung der Zellen vermieden. Es findet aber eine häufige Berührung zwischen den Komponenten der zelladhäsiven Beschichtung und den darauf sedimentierten Zellen statt.

Die beschriebenen Strömungsverhältnisse verhindern Zellstress und sorgen für eine homogene Versorgung mit Nährstoffen über die gesamte am Boden des Gerinnes aufwachsende Zellschicht.

### Ausführungsbeispiel 2;

In einen Mäander- Bioreaktor, beschrieben wie im Beispiel 1, mit der als Bodenfläche (6) der Gerinne (19) des Mäander- Bioreaktors ausgebildeten Zellkulturträger (20) aus dem Trägerkörper (21) und einer zelladhäsiven Beschichtung (22)mit darin angereicherten Anti- CD3 als aktivierende Antikörper, werden TILs aus zerkleinerten Gewebestücken gewonnen, wobei auch hier die Bedingungen der Bodenströmung in der Mäander- Perfusions-Kammer (4) nahe einer Froudezahl 0 eingehalten werden. Ausgangsmaterial dafür sind Gewebeteile, die bei operativer Entfernung eines Tumors, von Tumormetastasen oder von Tumorzelten befallenen Lymphknoten anfallen. Die zerkleinerten Gewebestücke werden durch den größer dimensionierten und mit einem steril verschließenden Stopfen eingefüllt und durch Schwenken des mit Medium gefüllten Mäander-Perfusions-Bioreaktors gleichmäßig auf dem Boden verteilt. Nach Verschließen des Einfüllstutzens wird die Kultivierung wie in Beispiel 1 in Gang gesetzt. Nach 3 bis 7 Tagen wachsen die in den tumorösen Gewebestücken enthaltenen Tumor-infiitrierten T-Lymphozyten (TIL) in Mengen aus. Je nach gewählten Medium und zugesetzten Supplementen werden die TIL in dieser Prozess-Phase aktiviert, durch weiteren Kontakt mit den anfangs noch vorhandenen Gemisch von mutierten Tumorzellen aus einem individuellen Tumorstück (gewonnen aus einem Tumor-Resektat) mutierten Tumorzellen spezifisch geprimt. In der Regel wachsen innerhalb einer Woche 1,5 bis 2 x 10⁹ TIL im Meander Perfusions-Bioreaktor mit 30 cm² Bodenfläche hoch. Nach Aufschütteln des Mäander-Perfusions-Bioreaktors werden die TIL durch den seitlichen Auslass-Stutzen (15), der mit einem Siebgewebe versehen ist, in ein steriles Gefäß oder in einen größer dimensionierten Mäander-Bioreaktor für die weitere Expansion. Die Gewebestücke, aus denen die TIL ausgewachsen sind, werden vollständig vom Siebgewebe des ersten Mäander-Bioreaktors zurückgehalten.

## Patentansprüche

1. Zellkulturträger (20) für Bioreaktoren zur Isolierung und Vermehrung von Zellen bestehend aus einem hydrophilen mit Niedertemperatur- Plasma behandelten Trägerkörper (21) mit einer zelladhäsiven Beschichtung (22), **dadurch gekennzeichnet, dass** in die zelladhäsiven Beschichtung (22) aktivierende Antikörper eingearbeitet sind.

2. Zellkulturträger (1) nach Anspruch 1, **dadurch gekennzeichnet**, das als aktivierende Antikörper Immunglobuline, β-Lymphozyten, Anti CD 16, Anti CD3, Anti CD28, Anti137, Anti CD39, Anti CD103 und/oder aus Kombinationen davon in die zelladhäsive Beschichtung (22) eingearbeitet sind.

3. Zellkulturträger (20) nach Anspruch 1, **dadurch gekennzeichnet, dass** die zelladhäsive Beschichtung (22) aus Flbronektin, RetroNectin, Collagen, Gelatine, Laminin, Chinitin und/oder aus Kombinationen davon besteht.

4. Zellkulturträger (20) nach Anspruch 1, **dadurch gekennzeichnet, dass** der Trägerkörper (21) aus Kunststoff, Glas, Metall oder keramischen Materialien besteht.

5. Zellkulturträger (20) nach Anspruch 4, **dadurch gekennzeichnet, dass** der Trägerkörper (21) aus einem klarem Polymermaterial besteht.

6. Zellkulturträger (20) nach Anspruch 1 bis 5, **dadurch gekennzeichnet, dass** der Zellkulturträger (20) in Bioreaktoren, Festbett- Bioreaktoren, Fließbett-Reaktoren, Walzen- Bioreaktoren, Hohlfaser- Bioreaktoren oder Mäander-Bioreaktoren oder als ein Teil von diesen zur Isolierung und Vermehrung von Zellen eingesetzt sind.
